# EUROPEAN PATENT APPLICATION

(11) **EP 4 822 010 A1**
(43) Date of publication of application: **14.10.2026**
(21) Application number: 26167290.1
(22) Date of filing: 24.03.2026
(51) Int. Cl.: C12M 1/42

(54) **CELL PUNCTURE DEVICE**

(30) Priority: 28.03.2025 JP 2025057081
(71) Applicant: Yokogawa Electric Corporation, Musashino-shi, Tokyo 180-8750 (JP)
(72) Inventor: KUWATA, Masahiro, Musashino-shi, Tokyo 180-8750 (JP)
(74) Representative: Stöckeler, Ferdinand

(57) **Abstract**

A cell puncture device 10 according to the present disclosure includes a needle 17a that punctures a cell S along a puncture direction, a driver 16 that drives the needle 17a, and a kinetic unit 65 that is coupled to the driver 16 and operates in the opposite direction to a change in momentum caused by movement of the driver 16 in the puncture direction, to change the momentum in the opposite direction.

## Description

### CROSS-REFERENCE TO RELATED APPLICATION

This application claims priority to Japanese Patent Application No. 2025-057081, filed on March 28, 2025, the entire contents of which are incorporated herein by reference.

### TECHNICAL FIELD

The present disclosure relates to a cell puncture device.

### BACKGROUND

In conventional research and applied development related to cells, technology for puncturing cells with needles is known, in order to accurately inject chemical solutions or the like into specific cells as samples, or to suck substances inside cells. For example, Patent Literature (PTL) 1 discloses that, in a device that uses a multi-barrel nano-pipette with at least two electrodes inside multiple barrels, one barrel draws out a cell inclusion and the other injects a substance into a cell.

### CITATION LIST

### Patent Literature

PTL 1: JP 6453300 B2

### SUMMARY

In the conventional technology described in PTL 1, for example, when a needle punctures a cell, a needle tip may vibrate inside a cell wall and cause damage to the interior of the cell wall. When vibration is large, it is assumed that the needle is repeatedly inserted into and removed from the cell, which also causes damage to the cell.

The present disclosure aims to provide a cell puncture device that can suppress vibration of a needle.
[1] A cell puncture device including:
   a needle configured to puncture a cell along a puncture direction;
   a driver configured to drive the needle; and
   a kinetic unit coupled to the driver, the kinetic unit being configured to operate in the opposite direction to a change in momentum caused by movement of the driver in the puncture direction, to change the momentum in the opposite direction.
   Such a cell puncture device can suppress vibration of the needle.
**[2]** In the cell puncture device described in [1] above, the operation of the kinetic unit in the opposite direction may be in conjunction with the movement of the driver in the puncture direction.
   This allows the cell puncture device to suppress vibration of the needle effectively.
[3] In the cell puncture device described in [1] or [2] above, the kinetic unit may include:
   a weight unit; and
   a hinge configured to move the weight unit in the opposite direction by the movement of the driver in the puncture direction.
   This allows the cell puncture device to suppress vibration of the needle more effectively.
[4] The cell puncture device described in [3] above may further include a coupler configured to couple the hinge to the driver. The weight unit may be connected to the hinge on the opposite side to the coupler, and may extend from the hinge.
   This allows the cell puncture device to suppress vibration of the needle more effectively.
[5] The cell puncture device described in [4] above may further include an arm configured to support the driver. The hinge may include a movable part, a first leaf spring configured to couple the coupler to the movable part on one side of the movable part, and a second leaf spring configured to couple the arm to the movable part on the other side of the movable part.
   This allows the cell puncture device to realize the hinge, which moves the weight unit in the opposite direction, with a simple configuration.
[6] In the cell puncture device described in any one of [3] to [5] above, the amount of movement of the weight unit in the opposite direction may be greater than the amount of the movement of the driver in the puncture direction.
   This allows the cell puncture device to suppress vibration of the needle more effectively.
[7] The cell puncture device described in any one of [3] to [6] above may include a movement unit configured to be moved in the puncture direction by the movement of the driver in the puncture direction. The weight of the weight unit may be less than that of the movement unit.
   This allows the cell puncture device to prevent the weight unit from excessively increasing in size.
[8] In the cell puncture device described in [5] above, the natural frequency of the weight unit may be 0.9 times or more and 1.1 times or less than the natural frequency of the arm.
   This can effectively suppress vibration of the needle.
[9] In the cell puncture device described in any one of [3] to [8] above, the natural frequency of the weight unit may be 0.9 times or more and 1.1 times or less than the natural frequency of the driver.
   This can effectively suppress vibration of the needle.
[10] In the cell puncture device described in any one of [3] to [9] above, the weight unit may have an attachment/detachment mechanism for at least one weight.
   This allows a user to adjust the magnitude of a change in the momentum of the weight unit in the opposite direction and the natural frequency of the weight unit by attaching or detaching the weight, thus effectively suppressing vibration of the needle.
[11] The cell puncture device according to any one of [3] to [10] above may further include a vibration damper configured to damp vibration of the weight unit.
   This allows the cell puncture device to suppress oscillation of the weight unit.
[12] In the cell puncture device described in any one of [1] to [11] above, the driver may drive the needle at a movement speed of 5 m/s or more.

This allows the cell puncture device to puncture the cell with the needle at a speed sufficient for the needle to penetrate the cell wall.

According to the present disclosure, it is possible to provide a cell puncture device that can suppress vibration of a needle.

### BRIEF DESCRIPTION OF THE DRAWINGS

In the accompanying drawings:
FIG. 1 is a schematic diagram illustrating an example of a configuration of a microscope system with a cell puncture device according to an embodiment of the present disclosure;
FIG. 2 is an enlarged view of an area near a second driver of the cell puncture device illustrated in FIG. 1;
FIG. 3 is a diagram illustrating the state of the second driver that has moved in a puncture direction from the state illustrated in FIG. 2;
FIG. 4 is a diagram illustrating details of a hinge of the cell puncture device of FIG. 1;
FIG. 5 is a graph illustrating the result of a comparative example; and
FIG. 6 is a graph illustrating the result of an example.

### DETAILED DESCRIPTION

### (Background and Problems)

The background and problems of conventional technology will be described in more detail.

In recent years, in research into biological systems and the like, there have been research into elucidating cell functions by injecting specific chemical solutions into cells and observing changes in the cells, and research into causing specific modifications to occur in specific cells by injecting, into the cells, chemical solutions to modify genes. In addition, there has also been applied development aiming at application to the production of chemical solutions or other substances. On the other hand, there have also been research into elucidating cell functions and applied development aiming at application to production by sucking and recovering, from specific cells, some of components that constitute the cells. In the above research and applied development, it is required, for example, to accurately inject the chemical solutions into the specific cells or to accurately suck the components from the specific cells, so it is desired that cell puncture devices can precisely puncture cells with needles.

PTL 1 discloses conventional technology for a method and device of injecting a chemical solution into a cell by controlling the position of a fine needle using a piezoelectric element, puncturing the cell by a needle tip, and controlling a voltage. Similarly, conventional technology for a method and device for sucking a substance from the interior of the cell is also disclosed.

In general, cells protect their interiors by cell walls, which are located in more external parts of the cells and cover the interiors. Therefore, in order to inject a chemical solution into a cell or to take out a substance from the interior of a cell by suction, a cell puncture device needs to insert a needle into the interior of the cell by controlling the position and speed of a needle tip so that the needle tip penetrates the cell wall. For this purpose, the cell puncture device preferably moves the needle at a high speed when the needle punctures the cell.

For example, in the case of a certain animal cell with a soft cell wall, the needle can penetrate the cell wall even if the cell puncture device moves the needle at a low speed when the needle punctures the cell. On the other hand, in the case of a certain plant cell with a hard cell wall, the needle cannot penetrate the cell wall unless the cell puncture device moves the needle at a high speed when the needle punctures the cell, which causes the problem of difficulty in inserting the needle tip into the cell.

As a method of moving the needle at a high speed as described above, a method of moving the needle using a piezoelectric element included in a driver that drives the needle is known. The piezoelectric element can cause an electrostrictive effect to occur inside itself by application of a voltage, and can expand and contract the piezoelectric element itself. The expansion and contraction of the piezoelectric element can respond more quickly than actuators such as general motors. Therefore, with the use of the piezoelectric element, the cell puncture device can cause the needle to puncture the cell at a high speed.

However, due to the high-speed movement of the needle using the piezoelectric element, there is a problem that the needle vibrates based on a reaction force when the needle is driven by the driver. For example, when part of the driver moves to puncture the cell, a reaction force occurs on a fixed side of the driver and causes vibration. In addition, when the needle punctures the cell, a force due to reaction is applied to a structure that supports the piezoelectric element. This causes the entire structure to move in the opposite direction to a puncture direction of the needle. As a result, there is also a problem that the needle vibrates after the puncture. When the needle vibrates after the puncture, the needle tip may vibrate inside the cell wall, and cause damage to the interior of the cell wall. When vibration is large, it is assumed that the needle is repeatedly inserted into and removed from the cell, which also causes damage to the cell. Therefore, for example, when a chemical solution is injected into the interior of the cell and its progress is observed, a cell survival rate is reduced due to damage to the cell caused by the vibration of the needle tip. As a result, there are cases in which the effects of the injected chemical solution cannot be evaluated.

In order to solve the problems described above, the present disclosure aims to provide a cell puncture device that can suppress vibration of a needle.

An embodiment of the present disclosure will be mainly described below with reference to the attached drawings. In the following description, x-, y-, and z-directions are with respect to the directions of the arrows in the drawings.

### (Configurations of Cell Puncture Device 10 and Microscope System 1)

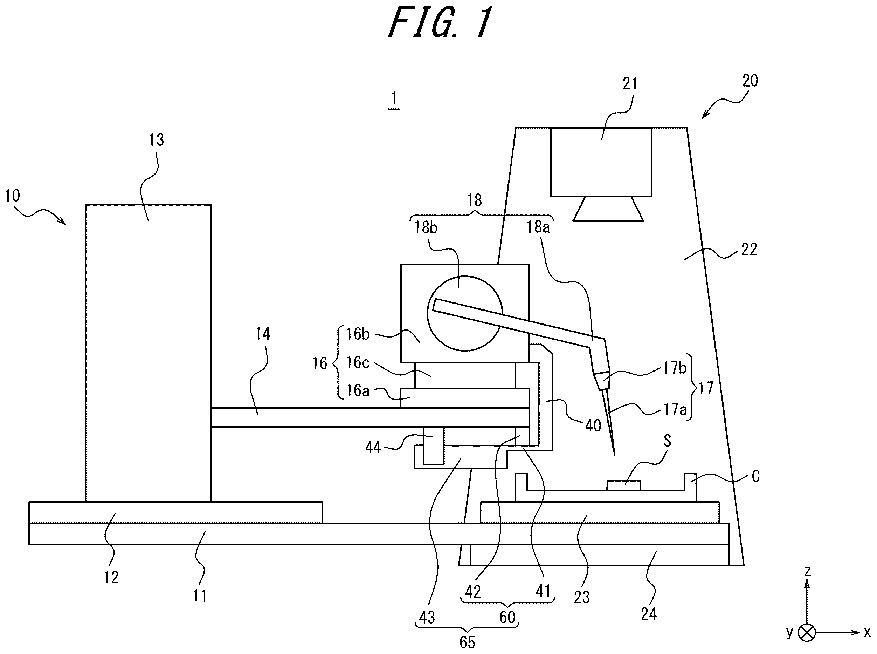

FIG. 1 is a schematic diagram illustrating an example of a configuration of a microscope system 1 with a cell puncture device 10 according to the embodiment of the present disclosure. With reference to FIGS. 1 to 4, an example of the configuration and functions of the microscope system 1 with the cell puncture device 10 according to the present embodiment will be mainly described. The microscope system 1 has the cell puncture device 10 and a microscope 20 that images a cell S to be punctured by a needle 17a of the cell puncture device 10.

The microscope 20 includes any microscope that can image the cell S. The microscope 20 includes, for example, a confocal microscope. The microscope 20 has any camera 21 that can image the cell S. The camera 21 constitutes an imager of the microscope 20. The microscope 20 has a pillar 22 that locates the camera 21 on one side of the cell S in the z-direction, so that the camera 21 can image the cell S from that side. The pillar 22 supports the camera 21 connected to an end of the pillar 22 on that side. The microscope 20 has a holder 23 that holds a petri dish C, on which the cell S is disposed, from the other side in the z-direction. The holder 23 is configured as a stage that is movable in two directions, the x- and y-directions. The microscope 20 has a support 24 that is located at an end of the pillar 22 on the other side and supports the holder 23, which holds the petri dish C.

The cell puncture device 10 has a first fixed part 11 that is disposed with respect to the imager, which images the cell S. The first fixed part 11 is fixed to, for example, the microscope 20, which images the cell S. The first fixed part 11 is configured in the shape of an arm and extends in the x-direction. The first fixed part 11 is disposed with respect to the holder 23 and the support 24 and is fixed to the microscope 20 by screwing one side of the first fixed part 11 in the x-direction onto the support 24 of the microscope 20. As an example, the first fixed part 11 is located between the holder 23 and the support 24, but the present disclosure is not limited thereto. The holder 23 may be located under the first fixed part 11. The first fixed part 11 is not limited to being screwed onto the support 24, but may be fixed to the microscope 20 in any other manner, such as by joining, fitting, or engaging. The cell puncture device 10 can be attached to the microscope 20 via the first fixed part 11.

The cell puncture device 10 has a base 12 that is connected to the other side of the first fixed part 11 in the x-direction and located on a surface of the first fixed part 11. The cell puncture device 10 has a first driver 13 that is supported by the first fixed part 11 and the base 12 so as to protrude from the base 12 to the positive side in the z-direction. The cell puncture device 10 has an arm 14 that extends from the first driver 13 to the positive side in the x-direction. The arm 14 is disposed with respect to the first fixed part 11 and the base 12. For example, the arm 14 is disposed in parallel with the first fixed part 11 and the base 12. The first driver 13 drives the arm 14 so that the arm 14 is movable in each of the x-, y-, and z-directions with respect to the first fixed part 11 and the base 12. For example, the first driver 13 may include an actuator that includes a ball screw and a motor to drive the arm 14.

The cell puncture device 10 has a second driver 16 supported by the arm 14. The second driver 16 is located on a surface of the arm 14 on the positive side in the z-direction. However, the disposition of the second driver 16 is not limited thereto. The second driver 16 may not be located on the surface of the arm 14 on the positive side in the z-direction. For example, the second driver 16 may be located on a surface of the arm 14 on the negative side in the z-direction.

The second driver 16 needs to move the needle 17a at a high speed in order to puncture the cell S. In order to achieve such high-speed movement of the needle 17a, the second driver 16 includes, for example, a piezoelectric element 16c that drives the needle 17a. The second driver 16 includes a second fixed part 16a connected to the arm 14, the piezoelectric element 16c that is connected to the second fixed part 16a and drives the needle 17a, and a first movable part 16b connected to the piezoelectric element 16c. The first movable part 16b is movable relative to the second fixed part 16a. The piezoelectric element 16c interposed between the first movable part 16b and the second fixed part 16a drives the needle 17a so that, for example, a needle tip of the needle 17a moves along the z-direction. The first movable part 16b causes the needle 17a to puncture the cell S by moving relative to the second fixed part 16a. More specifically, the first movable part 16b moves in the puncture direction with respect to the second fixed part 16a as the piezoelectric element 16c contracts, causing the needle 17a to puncture the cell S.

The cell puncture device 10 has a needle unit 17 that disposes, at a tip end, the needle 17a to puncture the cell S and that is driven by the first movable part 16b of the second driver 16. The needle unit 17 has the needle 17a that is driven by the second driver 16 and punctures the cell S, and a needle fixing part 17b that fixes the needle 17a.

The cell puncture device 10 has a needle support 18 that is connected to the needle fixing part 17b to dispose the needle unit 17 at a distal end. The needle support 18 has a support head 18a connected to the needle fixing part 17b, and a support head fixing part 18b connected to the support head 18a. The needle unit 17 can be operated by the second driver 16, by being supported by the support head 18a and by the support head 18a being attached to the support head fixing part 18b.

The needle 17a is driven by the second driver 16 to puncture the cell S along the puncture direction. In the present disclosure, the "puncture direction" refers to, for example, the direction from the positive size to the negative side in the z-direction.

The cell puncture device 10 may be configured so that by moving the base 12 in the x- or y-direction or rotating the base 12 relative to the first fixed part 11, the entire configuration including the first driver 13, the arm 14, the second driver 16, and the needle 17a can be withdrawn from the microscope 20. The movement of the base 12 in the x-direction or in the y-direction can be easily achieved, for example, by disposing linear guides, cross roller guides, and the like between the first fixed part 11 and the base 12. The rotation of the base 12 can be easily achieved, for example, by disposing ball bearings, cross roller bearings, and the like between the first fixed part 11 and the base 12.

The cell puncture device 10 may further have a fixing part (not illustrated in the drawings) between the first fixed part 11 and the base 12 in order to fix the relative movement of the base 12 in the x- or y-direction or the relative rotation of the base 12 with respect to the first fixed part 11. For example, the fixing part may fix the base 12 using a frictional force of a pin that can move in the z-direction so as to be pressed against the first fixed part 11 by a spring, or may fix the base 12 by a similar pin being engaged in a groove provided in the first fixed part 11. The pin can be easily lifted by disposing an operation unit to lift the pin to the positive side in the z-direction. The pin allows the base 12 to be moved and fixed relative to the first fixed part 11.

The cell puncture device 10 can precisely dispose the base 12 with respect to the first fixed part 11, due to the above-described configuration. The cell puncture device 10 can reduce fluctuations in the position of the needle 17a due to play of the base 12 with respect to the first fixed part 11.

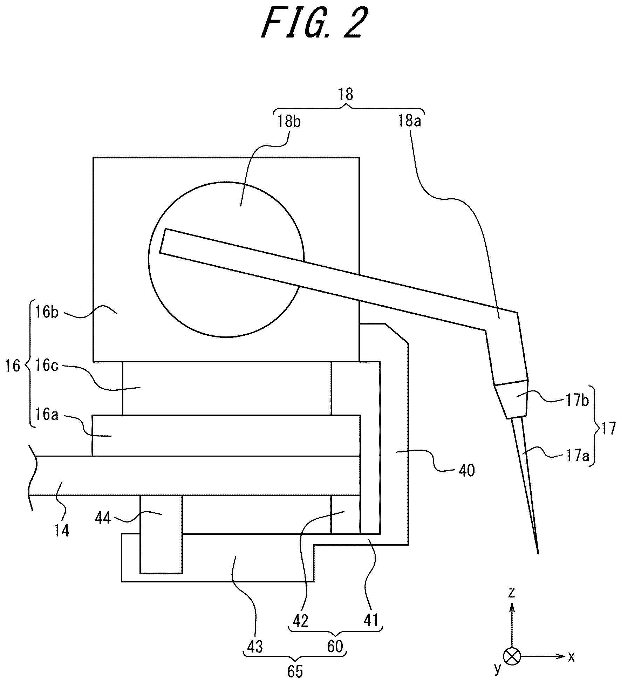

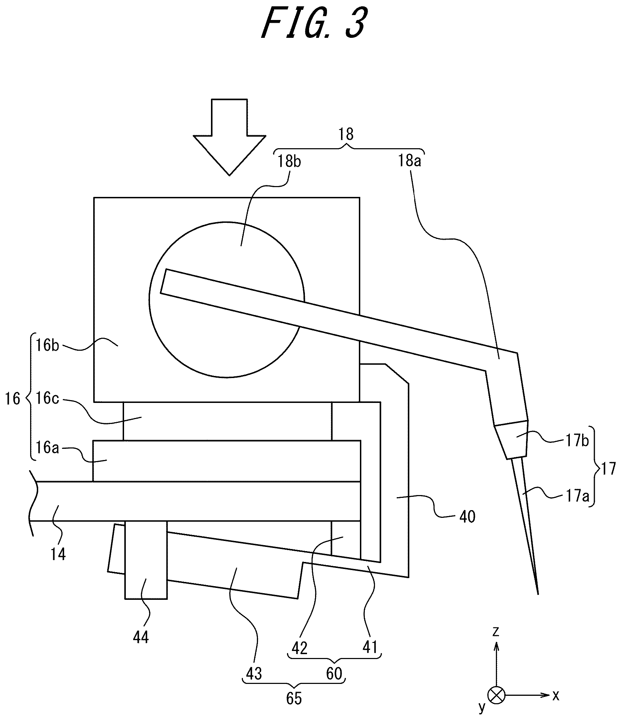

FIG. 2 is an enlarged view of an area near the second driver 16 of the cell puncture device 10. FIG. 3 is a diagram illustrating the state of the second driver 16 that has moved in the puncture direction from the state illustrated in FIG. 2. As illustrated in FIGS. 1 to 3, the cell puncture device 10 is provided with a kinetic unit 65 that is coupled to the second driver 16 and operates in the opposite direction to a change in momentum caused by movement of the second driver 16 in the puncture direction, to change the momentum in the opposite direction. Specifically, the cell puncture device 10 is provided with the kinetic unit 65 that is coupled to the second driver 16 and operates in the opposite direction to an increase in momentum caused by movement of the second driver 16 in the puncture direction, to increase the momentum in the opposite direction.

More specifically, as illustrated in FIGS. 2 and 3, the first movable part 16b moves in the puncture direction as the piezoelectric element 16c contracts. When the first movable part 16b moves in the puncture direction, the momentum of a movement unit in the puncture direction increases. In the present disclosure, the "movement unit" refers to a portion of the cell puncture device 10 that moves in the puncture direction by the movement of the second driver 16 (first movable part 16b) in the puncture direction. Specifically, the movement unit according to the present embodiment is constituted of, among components of the cell puncture device 10, the first movable part 16b and parts that are coupled to the first movable part 16b and move with the first movable part 16b in the puncture direction. Thus, the movement unit according to the present embodiment is constituted of the first movable part 16b, the support head fixing part 18b, the support head 18a, the needle fixing part 17b, and the needle 17a.

On the other hand, the kinetic unit 65 operates in the opposite direction (hereinafter simply described as "opposite direction") opposite to the puncture direction. In the present disclosure, the "opposite direction" corresponds to, for example, the direction from the negative side to the positive side in the z-direction. When the kinetic unit 65 operates in the opposite direction, the momentum of the kinetic unit 65 in the opposite direction increases.

The operation of the kinetic unit 65 in the opposite direction is in conjunction with the movement of the second driver 16 in the puncture direction. Specifically, the operation of the kinetic unit 65 in the opposite direction is in conjunction with the movement of the second driver 16 in the puncture direction so as to start approximately simultaneously with the start of the movement of the second driver 16 in the puncture direction. The kinetic unit 65 moves in the opposite direction as the second driver 16 moves in the puncture direction.

The kinetic unit 65 is provided with a weight unit 43 and a hinge 60 that moves the weight unit 43 in the opposite direction by the movement of the second driver 16 in the puncture direction. The weight unit 43 includes, for example, a block body formed of hard resin or metal.

Specifically, the hinge 60 is provided with a hinge movable part 41 and a hinge fixed part 42. The hinge fixed part 42 is fixed to a surface of the arm 14 on the negative side in the z-direction so as to project toward the negative side in the z-direction. The hinge movable part 41 extends in the x-direction over the hinge fixed part 42. The hinge movable part 41 is connected to the hinge fixed part 42 at a position between two ends in the x-direction. The hinge movable part 41 is connected to a tip end (an end on the negative side in the z-direction) of the hinge fixed part 42. The weight unit 43 is coupled to an end of the hinge movable part 41 on the negative side in the x-direction.

The cell puncture device 10 is provided with a coupler 40 that couples the hinge 60 to the second driver 16. The weight unit 43 is connected to the hinge 60 on the opposite side to the coupler 40, and extends from the hinge 60.

Specifically, the hinge 60 is disposed on the negative side in the z-direction relative to the second driver 16. The coupler 40 extends along the z-direction from one end connected to the first movable part 16b to the other end connected to the hinge movable part 41 of the hinge 60. The coupler 40 is connected to an end of the hinge movable part 41 on the positive side in the x-direction. The weight unit 43 is connected to an end of the hinge movable part 41 on the negative side in the x-direction, and extends toward the negative side in the x-direction.

As illustrated in FIGS. 2 and 3, when the piezoelectric element 16c contracts and the first movable part 16b moves in the puncture direction (the negative side in the z-direction), the coupler 40 connected to the first movable part 16b also moves in the puncture direction (the negative side in the z-direction). This also moves the end of the hinge movable part 41 on the positive side in the x-direction, which is connected to the coupler 40, in the puncture direction (the negative side in the z-direction). When the end of the hinge movable part 41 on the positive side in the x-direction moves in the puncture direction (the negative side in the z-direction), the hinge movable part 41 swings on the hinge fixed part 42 as a pivot. This causes the end of the hinge movable part 41 on the negative side in the x-direction to move in the opposite direction (the positive side in the z-direction). As a result, the weight unit 43 that is coupled to the end of the hinge movable part 41 on the negative side in the x-direction moves in the opposite direction (the positive side in the z-direction).

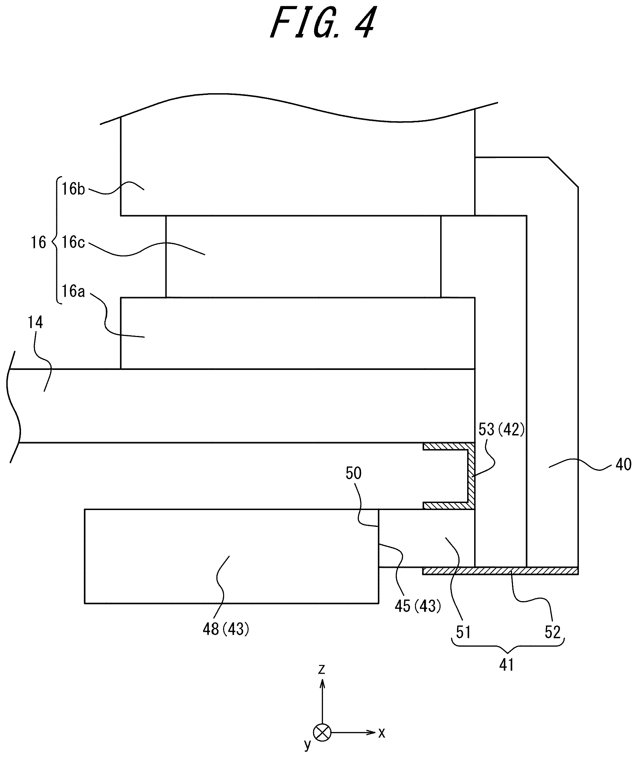

FIG. 4 is a diagram illustrating details of the hinge 60 of the cell puncture device 10 of FIG. 1. As illustrated in FIG. 4, the hinge movable part 41 has a second movable part 51 and a first leaf spring 52 that couples the second movable part 51 to the coupler 40 on one side of the second movable part 51 (in the present embodiment, the negative side of the second movable part 51 in the z-direction). The hinge fixed part 42 has a second leaf spring 53 that couples the second movable part 51 to the arm 14 on the other side of the second movable part 51 (in the present embodiment, the positive side of the second movable part 51 in the z-direction). The coupler 40 and the first leaf spring 52, and the second movable part 51 and the first leaf spring 52 may be coupled by fastening members such as screws, which are omitted from the drawing, or by welding, diffusion bonding, or other fusion bonding. The second movable part 51 and the second leaf spring 53, and the arm 14 and the second leaf spring 53 may be coupled by fastening members such as screws, which are omitted from the drawing, or by welding, diffusion bonding, or other fusion bonding.

The first leaf spring 52 is configured to be elastically deformable by bending in the z-direction. The second leaf spring 53 is configured to be elastically deformable by bending in the x-direction. The first and second leaf springs 52 and 53 are elastically deformed as the coupler 40 moves in the puncture direction. The elastic deformation of the first and second leaf springs 52 and 53 causes the hinge movable part 41 to swing on the hinge fixed part 42 as a pivot, and causes the weight unit 43 to move in the opposite direction (the positive side in the z-direction).

The weight of the weight unit 43 is less than that of the movement unit (in the present embodiment, the first movable part 16b, the support head fixing part 18b, the support head 18a, the needle fixing part 17b, and the needle 17a).

The cell puncture device 10 is configured so that the amount of movement of the weight unit 43 in the opposite direction when the second driver 16 moves in the puncture direction is greater than the amount of movement of the second driver 16 in the puncture direction. Specifically, the cell puncture device 10 is configured so that when the second driver 16 moves in the puncture direction, the amount of movement of the weight unit 43 in the opposite direction is approximately 600 µm as an example, while the amount of movement of the second driver 16 in the puncture direction is approximately 100 µm as an example. In other words, the cell puncture device 10 according to the present embodiment is configured so that when the second driver 16 moves in the puncture direction, the amount of movement of the weight unit 43 in the opposite direction is approximately six times, as an example, the amount of movement of the second driver 16 in the puncture direction.

The natural frequency of the weight unit 43 may be 0.9 times or more and 1.1 times or less than the natural frequency of the arm 14. In the present disclosure, the "natural frequency of the weight unit 43" refers to, for example, the frequency of the weight unit 43 in the z-direction. The natural frequency of the weight unit 43 may be calculated, for example, by measuring variations in the z-directional position of an end of the weight unit 43 on the negative side in the x-direction over time when the weight unit 43 is vibrated in the z-direction. In the present disclosure, the "natural frequency of the arm 14" refers to, for example, the frequency of the arm 14 in the z-direction. The natural frequency of the arm 14 may be calculated, for example, by measuring variations in the z-directional position of an end of the arm 14 on the positive side in the x-direction over time when the arm 14 is vibrated in the z-direction.

The natural frequency of the weight unit 43 may be 0.9 times or more and 1.1 times or less than the natural frequency of the second driver 16. In the present disclosure, the "natural frequency of the second driver 16" refers to, for example, the frequency of the first movable part 16b in the z-direction. The natural frequency of the second driver 16 may be calculated, for example, by measuring variations in the z-directional position of an end of the first movable part 16b on the positive side in the z-direction over time when the first movable part 16b is vibrated in the z-direction.

As illustrated in FIG. 4, the weight unit 43 has an attachment/detachment mechanism 45 for at least one weight 48. Specifically, the weight unit 43 according to the present embodiment has the single weight 48 and the attachment/detachment mechanism 45 provided on the weight 48. The attachment/detachment mechanism 45 is provided at an end of the weight 48 on the positive side in the x-direction. The weight 48 is detachably connected to a connection part 50 of the hinge 60 via the attachment/detachment mechanism 45. The connection part 50 of the hinge 60 is provided at an end of the second movable part 51 on the negative side in the x-direction. The attachment/detachment mechanism 45 may include, for example, a fastening member that screws the weight 48 to the second movable part 51. The attachment/detachment mechanism 45 may be a mechanism that mechanically locks the weight 48 to the second movable part 51, for example, by allowing the weight 48 to be screwed, engaged, moored, or fitted to the connection part 50 of the second movable part 51. The weight unit 43 may further have two or more weights 48 and attachment/detachment mechanisms 45 for attaching/detaching these weights 48 to/from each other.

As illustrated in FIGS. 1 to 3, the cell puncture device 10 is provided with two vibration dampers 44 that damp vibration of the weight unit 43. The vibration dampers 44 may include, for example, damping rubbers or dampers that convert part of kinetic energy into thermal energy through deformation.

The vibration dampers 44 are disposed with respect to the arm 14 so as to be in contact with the weight unit 43. The two vibration dampers 44 are disposed on both sides in the y-direction with respect to the weight unit 43, so as to sandwich the weight unit 43 from both sides in the y-direction. Not limited thereto, only one vibration damper 44 may be disposed with respect to the weight unit 43, or three or more vibration dampers 44 may be disposed. The vibration dampers 44 may be disposed between the arm 14 and the weight unit 43 so as to fill clearance between the arm 14 and the weight unit 43 along the z-direction.

The second driver 16 drives the needle 17a at a movement speed of 5 m/s or more. Specifically, in the cell puncture device 10 according to the present embodiment, the first movable part 16b moves in the puncture direction with respect to the second fixed part 16a so that the movement speed of the needle 17a is 5 m/s or more, by expanding or contracting the piezoelectric element 16c.

### (Effects)

The cell puncture device 10 according to the embodiment described above can suppress vibration of the needle 17a. The cell puncture device 10 is provided with the kinetic unit 65 that is coupled to the second driver 16 and operates in the opposite direction to a change in momentum caused by movement of the second driver 16 in the puncture direction, to change the momentum in the opposite direction. When the second driver 16 moves in the puncture direction, the momentum of the movement unit (in the present embodiment, the first movable part 16b, the support head fixing part 18b, the support head 18a, the needle fixing part 17b, and the needle 17a) in the puncture direction increases. On the other hand, when the kinetic unit 65 operates in the opposite direction, the momentum of the kinetic unit 65 in the opposite direction increases. Here, the momentum of the entire cell puncture device 10 is conserved. Therefore, even when the momentum of the movement unit in the puncture direction increases due to the movement of the second driver 16 in the puncture direction, the cell puncture device 10 can suppress a change in the momentum of the entire cell puncture device 10 by increasing the momentum of the kinetic unit 65 in the opposite direction. This allows the cell puncture device 10 to suppress the generation of a reaction force when the needle 17a is driven by the second driver 16, and to suppress vibration of the needle 17a when the needle 17a punctures the cell S. Thus, for example, when the needle 17a punctures the cell S, it is possible to suppress vibration of the tip of the needle 17a inside the cell wall, thereby preventing damage to the interior of the cell wall. For example, it is also possible to prevent the needle 17a from being inserted into or removed from the cell S repeatedly.

The operation of the kinetic unit 65 in the opposite direction is in conjunction with the movement of the second driver 16 in the puncture direction. Therefore, the kinetic unit 65 can operate in the opposite direction in conjunction with the timing of an increase in the momentum of the movement unit in the puncture direction due to the movement of the second driver 16 in the puncture direction. This allows the cell puncture device 10 to effectively suppress a change in the momentum of the entire cell puncture device 10 and thus to effectively suppress vibration of the needle 17a.

The kinetic unit 65 is provided with the weight unit 43 and the hinge 60 that moves the weight unit 43 in the opposite direction by the movement of the second driver 16 in the puncture direction. Therefore, when the second driver 16 moves in the puncture direction, the weight unit 43 is moved in the opposite direction by the hinge 60. Accordingly, even when the momentum of the movement unit in the puncture direction increases due to the movement of the second driver 16 in the puncture direction, the cell puncture device 10 can suppress a change in the momentum of the entire cell puncture device 10 by increasing the momentum of the weight unit 43 in the opposite direction. In other words, the cell puncture device 10 can suppress vibration of the needle 17a more effectively because the kinetic unit 65 is provided with the weight unit 43 and the hinge 60.

The cell puncture device 10 is provided with the coupler 40 that couples the hinge 60 to the second driver 16. The weight unit 43 is connected to the hinge 60 on the opposite side to the coupler 40, and extends from the hinge 60. Since the cell puncture device 10 is provided with the coupler 40, a driving force that moves the weight unit 43 in the opposite direction can be transmitted to the hinge 60 via the coupler 40 when the second driver 16 moves in the puncture direction. Since the weight unit 43 is connected to the hinge 60 on the opposite side to the coupler 40 and extends from the hinge 60, the cell puncture device 10 can move the weight unit 43 in the opposite direction more effectively by utilizing the driving force transmitted to the hinge 60 via the coupler 40. Therefore, the cell puncture device 10 can suppress vibration of the needle 17a more effectively due to the provision of the coupler 40 and the weight unit 43 connected to the hinge 60 on the opposite side to the coupler 40 and extending from the hinge 60.

In the cell puncture device 10, the hinge 60 has the second movable part 51, the first leaf spring 52 that couples the coupler 40 to the second movable part 51 on one side of the second movable part 51, and the second leaf spring 53 that couples the arm 14 to the second movable part 51 on the other side of the second movable part 51. This allows the cell puncture device 10 to realize the hinge 60 that moves the weight unit 43 in the opposite direction, with a simple configuration.

The natural frequency of the weight unit 43 may be 0.9 times or more and 1.1 times or less than the natural frequency of the arm 14. This allows the cell puncture device 10 to bring the natural frequency of the weight unit 43 closer to that of the arm 14. Here, the cell puncture device 10 may not be able to completely suppress a change in the momentum of the entire cell puncture device 10, and the arm 14 may remain in a vibrating state for some time due to a reaction force generated by a puncture operation of the needle 17a. In this case, the needle 17a also remains in a vibrating state. Accordingly, by setting the natural frequency of the weight unit 43 to be 0.9 times or more and 1.1 times or less than the natural frequency of the arm 14, the cell puncture device 10 can reduce the vibration of the arm 14 using vibration of the weight unit 43 generated by the weight unit 43 being moved in the opposite direction, and converge the vibration of the arm 14 more quickly. This allows the cell puncture device 10 to suppress the vibration of the needle 17a effectively.

The natural frequency of the weight unit 43 may be 0.9 times or more and 1.1 times or less than the natural frequency of the second driver 16. This allows the cell puncture device 10 to bring the natural frequency of the weight unit 43 closer to that of the second driver 16. Here, the cell puncture device 10 may not be able to completely suppress a change in the momentum of the entire cell puncture device 10, and the second driver 16 (in the present embodiment, especially the first movable part 16b) may remain in a vibrating state for some time due to a reaction force generated by a puncture operation of the needle 17a. In this case, the needle 17a also remains in a vibrating state. Accordingly, by setting the natural frequency of the weight unit 43 to be 0.9 times or more and 1.1 times or less than the natural frequency of the second driver 16, the cell puncture device 10 can reduce the vibration of the second driver 16 using vibration of the weight unit 43 generated by the weight unit 43 being moved in the opposite direction, and converge the vibration of the second driver 16 more quickly. This allows the cell puncture device 10 to suppress the vibration of the needle 17a effectively.

The natural frequency of the weight unit 43 can be varied by varying the weight of the weight unit 43. The natural frequency of the weight unit 43 can be varied by varying the elasticity of the hinge 60. Specifically, in the present embodiment, the natural frequency of the weight unit 43 can be varied by varying the elastic force of the first and second leaf springs 52 and 53 of the hinge 60.

The weight of the weight unit 43 is less than that of the movement unit (in the present embodiment, the first movable part 16b, the support head fixing part 18b, the support head 18a, the needle fixing part 17b, and the needle 17a). This allows the cell puncture device 10 to prevent the weight unit 43 from excessively increasing in size.

On the other hand, when the weight of the weight unit 43 is light, the momentum of the weight unit 43 in the opposite direction may be less likely to increase, and the cell puncture device 10 may be less likely to inhibit a change in the momentum of the entire cell puncture device 10. Accordingly, the cell puncture device 10 is configured so that the amount of movement of the weight unit 43 in the opposite direction when the second driver 16 moves in the puncture direction is greater than the amount of movement of the second driver 16 in the puncture direction. This makes it easier for the cell puncture device 10 to increase the momentum of the weight unit 43 in the opposite direction. As a result, the cell puncture device 10 can effectively suppress a change in the momentum of the entire cell puncture device 10, and thus effectively suppress vibration of the needle 17a.

As described above, the cell puncture device 10 according to the present embodiment is configured so that when the second driver 16 moves in the puncture direction, the amount of movement of the weight unit 43 in the opposite direction is approximately 600 µm, while the amount of movement of the second driver 16 in the puncture direction is approximately 100 µm. Therefore, in the cell puncture device 10 according to the present embodiment, the amount of movement of the weight unit 43 in the opposite direction is approximately six times the amount of movement of the second driver 16 in the puncture direction. Therefore, in the cell puncture device 10 according to the present embodiment, the momentum of the weight unit 43 in the opposite direction that is increased by the weight unit 43 moving in the opposite direction can be approximately six times larger than the momentum of the weight unit 43 in the opposite direction that is increased when the weight unit 43 is moved by the same amount as the amount of movement of the second driver 16 in the puncture direction. This allows the cell puncture device 10 according to the present embodiment to effectively suppress a change in the momentum of the entire cell puncture device 10 and thus to effectively suppress vibration of the needle 17a.

The weight unit 43 has the attachment/detachment mechanism 45 for at least one weight 48. Therefore, a user can vary the weight and/or length of the weight unit 43 by detaching the weight 48 and replacing the weight 48 with a weight 48 having a different weight and/or length. This allows the user to adjust the magnitude of a change in the momentum of the weight unit 43 in the opposite direction as the weight unit 43 is moved in the opposite direction. The user can also adjust the natural frequency of the weight unit 43 by attaching or detaching the weight 48. In other words, the user can adjust the magnitude of a change in the momentum of the weight unit 43 in the opposite direction and the natural frequency of the weight unit 43 by attaching or detaching the weight 48, thus effectively suppressing vibration of the needle 17a.

The cell puncture device 10 is provided with the vibration dampers 44 that damp vibration of the weight unit 43. Here, depending on the natural frequency of the weight unit 43, an operation of the second driver 16 may cause the weight unit 43 to oscillate, which may increase vibration of the needle 17a. The provision of the vibration dampers 44, which damp the vibration of the weight unit 43, allows the cell puncture device 10 to suppress such oscillation.

The second driver 16 drives the needle 17a at a movement speed of 5 m/s or more. The cell puncture device 10 can thereby puncture the cell S with the needle 17a at a speed sufficient for the needle 17a to penetrate the cell wall, even when the cell S is a plant cell having a hard cell wall.

### (Variations)

While the present disclosure is described based on the drawings and embodiment, it should be noted that various modifications and revisions can be made by those skilled in the art based on the present disclosure. Accordingly, such modifications and revisions are included within the scope of the present disclosure. For example, the functions included in each configuration or each operation can be rearranged in a logically consistent manner, and multiple configurations or operations can be combined into one or divided.

For example, the shape, pattern, size, disposition, orientation, type, number, and the like of each component described above are not limited to those illustrated in the above description and drawing. The shape, pattern, size, disposition, orientation, type, number, and the like of each component may be configured arbitrarily, as long as the function can be realized. Each component of the cell puncture device 10 and the microscope system 1 illustrated in the drawings is a functional concept, and the specific form of each component is not limited to that illustrated in the drawings.

The above embodiment describes a configuration in which the movement of the kinetic unit 65 in the opposite direction is in conjunction with the movement of the second driver 16 in the puncture direction, but the present disclosure is not limited thereto. The movement of the kinetic unit 65 in the opposite direction may not be in conjunction with the movement of the second driver 16 in the puncture direction.

The above embodiment describes a configuration in which the kinetic unit 65 is provided with the weight unit 43 and the hinge 60 that moves the weight unit 43 in the opposite direction by the movement of the second driver 16 in the puncture direction, but the present disclosure is not limited thereto. For example, the kinetic unit 65 may be configured to move the weight unit 43 in the opposite direction by means other than the hinge 60.

The above embodiment describes a configuration in which the cell puncture device 10 is provided with the coupler 40 that couples the hinge 60 to the second driver 16, and the weight unit 43 is connected to the hinge 60 on the opposite side to the coupler 40 and extends from the hinge 60, but the present disclosure is not limited thereto. For example, the cell puncture device 10 may not be provided with the coupler 40. In this case, the hinge 60 may be directly coupled to the second driver 16.

The above embodiment describes a configuration in which the cell puncture device 10 is provided with the arm 14 that supports the driver, and the hinge 60 has the movable part, the first leaf spring 52 that connects the coupler 40 and the movable part on one side of the movable part, and the second leaf spring 53 that connects the arm 14 and the movable part on the other side of the movable part, but the present disclosure is not limited thereto. The hinge 60 may not be provided with the first and second springs 52 and 53, as long as the movement of the second driver 16 in the puncture direction allows the weight unit 43 to move in the opposite direction.

The above embodiment describes a configuration in which the natural frequency of the weight unit 43 may be 0.9 times or more and 1.1 times or less than the natural frequency of the arm 14. The above embodiment describes a configuration in which the natural frequency of the weight unit 43 may be 0.9 times or more and 1.1 times or less than the natural frequency of the second driver 16. However, the natural frequency of the weight unit 43 may be set in a range other than these. For example, the natural frequency of the weight unit 43 may be set to be less than 0.9 times the natural frequency of the arm 14. For example, the natural frequency of the weight unit 43 may be set to be less than 0.9 times the natural frequency of the second driver 16.

The above embodiment describes a configuration in which the weight unit 43 has the attachment/detachment mechanism 45 for the single weight 48, but the present disclosure is not limited thereto. For example, the weight unit 43 may have the function of attaching and detaching a plurality of weights 48. In this case, the user can adjust the amount of a change in the momentum of the weight unit 43 and the natural frequency of the weight unit 43 more precisely. The weight unit 43 may not have the function of attaching and detaching the weight 48. For example, the weight unit 43 may be constituted of a weight 48 that is integral with the hinge 60.

The above embodiment describes a configuration in which the cell puncture device 10 is provided with the vibration dampers 44 that damp vibration of the weight unit 43, but the present disclosure is not limited thereto. The cell puncture device 10 may not be provided with the vibration dampers 44.

The above embodiment describes a configuration in which the vibration dampers 44 are disposed with respect to the arm 14 so as to be in contact with the weight unit 43, but the present disclosure is not limited thereto. The vibration dampers 44 may not be in contact with the weight unit 43 as long as the vibration dampers 44 can damp vibration of the weight unit 43. For example, the vibration dampers 44 may be disposed with respect to the arm 14 so as not to be in contact with the weight unit 43, but in contact only with the second movable part 51 of the hinge 60.

The above embodiment describes a configuration in which the second driver 16 drives the needle 17a at a movement speed of 5 m/s or more, but the present disclosure is not limited thereto. The second driver 16 may drive the needle 17a at a movement speed of less than 5 m/s.

The above embodiment describes a configuration in which the weight of the weight unit 43 is less than that of the movement unit, but the present disclosure is not limited thereto. The weight of the weight unit 43 may be the same as that of the movement unit, or may be greater than that of the movement unit. By varying the weight of the weight unit 43, the cell puncture device 10 can adjust the amount of a change in the momentum of the weight unit 43 in the opposite direction due to the weight unit 43 moving in the opposite direction. Therefore, the cell puncture device 10 may be able to inhibit a change in the momentum of the entire cell puncture device 10 with a smaller amount of movement of the weight unit 43, for example, by making the weight of the weight unit 43 greater than that of the movement unit. For example, depending on the type of the cell S to be punctured, leaving slight vibration on the needle 17a after the needle 17a is punctured may make it easier for the needle 17a to enter the cell S because the needle 17a continuously moves to puncture the cell S. In such a case, the cell puncture device 10 can control the amount of vibration of the needle 17a after a puncture operation, for example, by reducing the weight of the weight unit 43 to some extent, to leave slight vibration on the needle 17a.

The shape, size, and weight of the support head fixing part 18b, the support head 18a, the needle fixing part 17b, and the needle 17a coupled to the first movable part 16b can be changed as appropriate depending on the application of the cell puncture device 10. Alternatively, components other than these may be coupled to the first movable part 16b and used. In such a case, the user can still adjust the amount of vibration of the needle 17a after puncture by varying the weight of the weight unit 43.

The above embodiment describes a configuration in which the cell puncture device 10 is configured so that the amount of movement of the weight unit 43 in the opposite direction when the second driver 16 moves in the puncture direction is greater than the amount of movement of the second driver 16 in the puncture direction, but the present disclosure is not limited thereto. The cell puncture device 10 may be configured so that the amount of movement of the weight unit 43 in the opposite direction when the second driver 16 moves in the puncture direction is the same as the amount of movement of the second driver 16 in the puncture direction, or is less than the amount of movement of the second driver 16 in the puncture direction. The amount of movement of the weight unit 43 in the opposite direction when the second driver 16 moves in the puncture direction can be adjusted, for example, by changing the positional relationship between the hinge movable part 41, the hinge fixed part 42, and the coupler 40. This is the same as leverage and corresponds to the fact that changing the positional relationship between a fulcrum and a point of application changes the ratio of the amount of movement of a point of effort to the amount of movement of the point of application.

### EXAMPLES

The cell puncture device 10 according to the present embodiment will be described in more detail below using examples, but the present disclosure is not limited to the following examples. The numerical values described in the examples are just examples and do not limit the scope of the present disclosure. The scope of the present disclosure should be defined solely on the basis of the claims. In the following description, the same components as in the embodiment are denoted by the same reference numerals, and redundant descriptions are omitted.

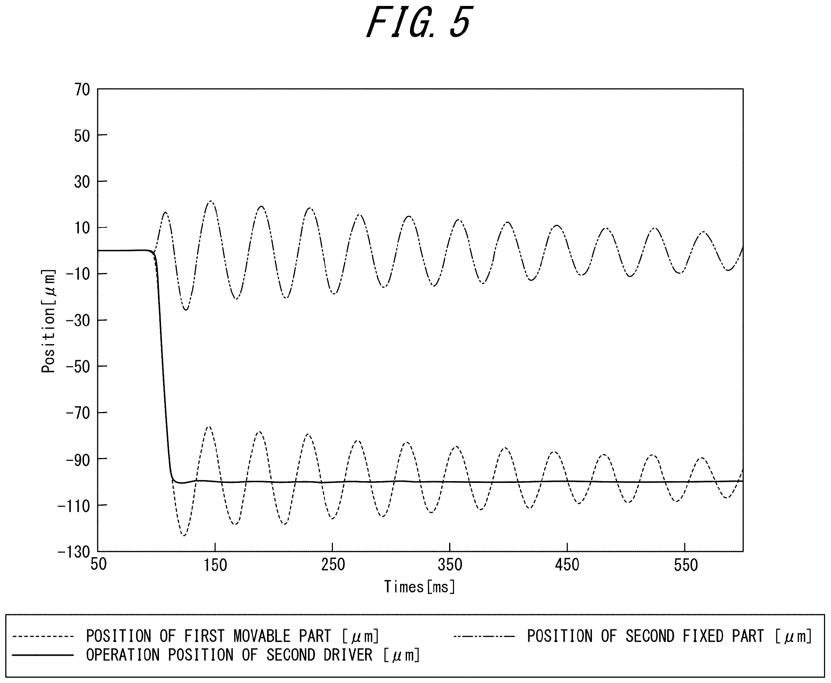

First, a comparative example is described with reference to FIG. 5. FIG. 5 is a graph illustrating the experimental result of a puncture operation of a needle using a conventional cell puncture device without a kinetic unit.

In FIG. 5, the positive direction of the vertical axis corresponds to a withdrawal direction, and the negative direction of the vertical axis corresponds to a puncture direction. In FIG. 5, the operation position of a second driver illustrated in the solid line is a position relative to an arm. The positions of other components are absolute positions (or positions relative to a portion functioning as a base of the cell puncture device, such as a support).

As illustrated in FIG. 5, in the comparative example, when the second driver moved in the puncture direction (see solid line in FIG. 5), a reaction force generated by the movement of the second driver in the puncture direction caused a second fixed part fixed to the arm to vibrate (see chain double-dashed line in FIG. 5). Due to this effect, a first movable part also vibrated (see dashed line in FIG. 5). Therefore, when the cell puncture device according to the comparative example was used, the needle vibrated and punctured a cell repeatedly.

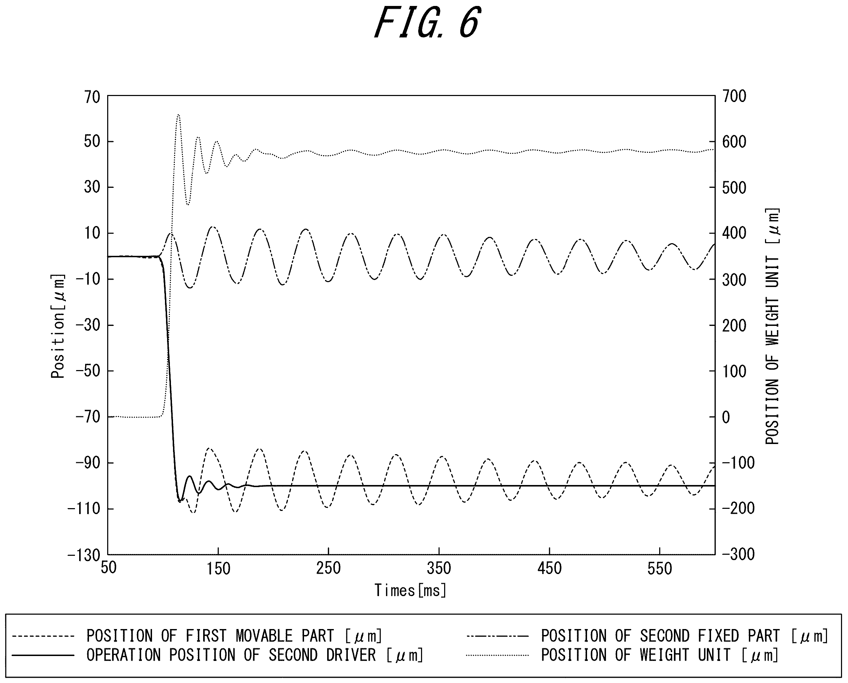

Next, an example is described with reference to FIG. 6. FIG. 6 is a graph illustrating the experimental result of a puncture operation of the needle 17a using the cell puncture device 10 according to the present embodiment with the kinetic unit 65.

In FIG. 6, the positive direction of the vertical axis corresponds to a withdrawal direction, and the negative direction of the vertical axis corresponds to the puncture direction. In FIG. 6, the operation position of the second driver 16 illustrated in the solid line is a position relative to the arm 14. The positions of other components are absolute positions (or positions relative to a portion functioning as a base of the cell puncture device 10, such as the support 24).

As illustrated in FIG. 6, in the example, the second driver 16 first moved in the puncture direction (see solid line in FIG. 6). As the first movable part 16b was moved in the puncture direction, the coupler 40 coupled to the first movable part 16b was also moved in the puncture direction, and accordingly the hinge 60 moved the weight unit 43 in the opposite direction (see dotted line in FIG. 6). As a result, even when the momentum of the first movable part 16b, the support head fixing part 18b, the support head 18a, the needle fixing part 17b, and the needle 17a in the puncture direction increased due to the movement of the first movable part 16b in the puncture direction, a change in the momentum of the entire cell puncture device 10 was suppressed by increasing the momentum of the weight unit 43 in the opposite direction. As a result, a reaction force causing vibration of the arm 14 was reduced, and thus vibration of the arm 14 was reduced. Therefore, in this example, the amount of vibration of the second fixed part 16a (see chain double-dashed line in FIG. 6) was smaller than that of the second fixed part in the comparative example (see chain double-dashed line in FIG. 5). Accordingly, in this example, the amount of vibration of the first movable part 16b (see dashed line in FIG. 6) was smaller than that of the first movable part in the comparative example (see dashed line in FIG. 6). From this result, it was confirmed that the cell puncture device 10 according to the present embodiment could suppress vibration of the needle 17a.

Here, as illustrated in FIG. 6, vibration of the weight unit 43 was damped and reduced at an early stage. This is because the vibration dampers 44 connected to the weight unit 43 converted vibration generated in the weight unit 43 into thermal energy, to damp the vibration.

Therefore, the second driver 16 vibrated immediately after moving in the puncture direction because the second driver 16 was affected by the vibration of the weight unit 43 via the coupler 40, but vibration of the second driver 16 was damped and reduced in the same manner as the vibration generated in the weight unit 43. The position of the second driver 16 is important for determining the position of the needle tip to puncture the cell, so preferably has minimal fluctuation. The cell puncture device 10 according to the present embodiment damps vibration of the weight unit 43 using the vibration dampers 44 coupled to the weight unit 43, thereby suppressing vibration occurring in the second driver 16 and enabling the needle 17a to puncture at a precise position.

## Claims

1. A cell puncture device comprising:
a needle configured to puncture a cell along a puncture direction;
a driver configured to drive the needle; and
a kinetic unit coupled to the driver, the kinetic unit being configured to operate in an opposite direction to a change in momentum caused by movement of the driver in the puncture direction, to change the momentum in the opposite direction.

2. The cell puncture device according to claim 1, wherein an operation of the kinetic unit in the opposite direction is in conjunction with the movement of the driver in the puncture direction.

3. The cell puncture device according to claim 1 or 2, wherein the kinetic unit comprises:
a weight unit; and
a hinge configured to move the weight unit in the opposite direction by the movement of the driver in the puncture direction.

4. The cell puncture device according to claim 3, further comprising a coupler configured to couple the hinge to the driver,
wherein the weight unit is connected to the hinge on an opposite side to the coupler and extends from the hinge.

5. The cell puncture device according to claim 4, further comprising an arm configured to support the driver,
wherein the hinge comprises:
a movable part;
a first leaf spring configured to couple the coupler to the movable part on one side of the movable part; and
a second leaf spring configured to couple the arm to the movable part on the other side of the movable part.

6. The cell puncture device according to claim 3, wherein an amount of movement of the weight unit in the opposite direction is greater than an amount of the movement of the driver in the puncture direction.

7. The cell puncture device according to claim 3, comprising a movement unit configured to be moved in the puncture direction by the movement of the driver in the puncture direction,
wherein a weight of the weight unit is less than a weight of the movement unit.

8. The cell puncture device according to claim 5, wherein a natural frequency of the weight unit is 0.9 times or more and 1.1 times or less than a natural frequency of the arm.

9. The cell puncture device according to claim 3, wherein a natural frequency of the weight unit is 0.9 times or more and 1.1 times or less than a natural frequency of the driver.

10. The cell puncture device according to claim 3, wherein the weight unit has an attachment/detachment mechanism for at least one weight.

11. The cell puncture device according to claim 3, further comprising a vibration damper configured to damp vibration of the weight unit.

12. The cell puncture device according to claim 1 or 2, wherein the driver is configured to drive the needle at a movement speed of 5 m/s or more.
